(19) Europäisches Patentamt
European Patent Office
Office européen des brevets

(11) Veröffentlichungsnummer: **0 139 907**
**B1**

(12) **EUROPÄISCHE PATENTSCHRIFT**

(45) Veröffentlichungstag der Patentschrift: **06.02.91**

(51) Int. Cl.$^5$: **C 07 J 1/00, C 12 P 33/12**

(21) Anmeldenummer: **84109136.6**

(22) Anmeldetag: **01.08.84**

(54) **Verfahren zur Herstellung von Estran-Derivaten.**

(30) Priorität: **26.08.83 DE 3331288**

(43) Veröffentlichungstag der Anmeldung:
**08.05.85 Patentblatt 85/19**

(45) Bekanntmachung des Hinweises auf die Patenterteilung:
**06.02.91 Patentblatt 91/06**

(84) Benannte Vertragsstaaten:
**AT BE CH DE FR GB IT LI LU NL SE**

(56) Entgegenhaltungen:
**DE-A-1 223 372**
**US-A-3 179 674**
**US-A-3 243 355**
**US-A-3 766 224**

**A.I. LASKIN et al.: J. Org. Chem. 29, 1333 (1964)**
**H.L. HOLLAND et al.: Can. J. Chem. 63, 1121 (1985)**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Neef, Günter, Dr.**
**Darmstädter Strasse 9**
**D-1000 Berlin 15 (DE)**
Erfinder: **Petzoldt, Karl, Dr.**
**Flachsweg 10**
**D-1000 Berlin 38 (DE)**
Erfinder: **Sauer, Gerhard, Dr.**
**Königsbacher Zeile 47 A**
**D-1000 Berlin 28 (DE)**
Erfinder: **Hofmeister, Helmut, Dr.**
**Weislingenstrasse 4**
**D-1000 Berlin 28 (DE)**
Erfinder: **Wiechert, Rudolf, Prof. Dr.**
**Petzower Strasse 8 A**
**D-1000 Berlin 39 (DE)**
Erfinder: **Wieglepp, Heinz**
**Herbststrasse 59**
**D-1000 Berlin 51 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

**Beschriebung**

Die Erfindung betrifft ein Verfahren zur Herstellung von neuen Estran-Derivaten der allgemeinen Formel I

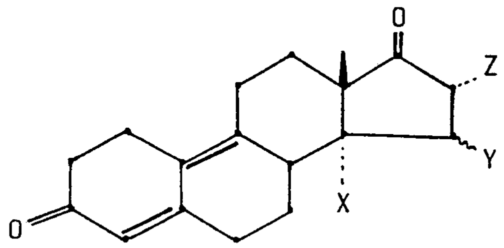

(I),

worin einer der Substituenten X oder Z ein Wasserstoffatom bedeutet und der andere gemeinsam mit Y eine Kohlenstoff-Kohlenstoffbindung darstellt, oder worin die Substituenten X und Z Wasserstoffatome darstellen und Y eine α-ständige Hydroxygruppe oder eine β-ständige Alkylgruppe mit bis zu 6 Kohlenstoffatomen darstellt, welches dadurch gekennzeichnet ist, daß man 4,9-Estradien-3,17-dion mit einer Pilzkultur von Fusarium oxysporum, Glomerella glycines, Glomerella fusaroides, Absidia coerulea fermentiert und gewünschtenfalls das erhaltene 15α-Hydroxy-4,9-erstradien-3,17-dion in den Trimethylessigsäureester oder den Phenylsulfenylester überführt, die Trimethylacetylgruppe oder die Phenylsulfenylgruppe mittels Basen abspaltet sowie gegebenenfalls das erhaltene 4,9,15-Estratrien-3,17-dion mit einer Kupferalkylverbindung der allgemeinen Formel II oder III

$R_2CuLi$ (II) oder $R_2Cu(CN)Li_2$ (III),

worin R einen Alkylrest mit bis zu 6 Kohlenstoffatomen darstellt, alkyliert.

Es ist bekannt, daß die Herstellung von 15ß-Alkylsteroiden auf chemischen Wege erhebliche Schwierigkeiten bereitet (US—A—3,766,224). Es wurde nun gefunden, daß man das 4,9-Estradien-3,17-dion mit den vorstehend genannten Stämmen unter Erzielung ausreichender Ausbeuten an Verfahrensprodukt in der 15α-Position hydroxylieren kann.

Dies ist für den Fachmann überraschend. Zwar ist aus der US—A—3,243,355 vorbekannt, daß man 19-Nor-4-androsten-3,17-dion unter Erzielung befriedigender Ausbeuten in der 15α-Position hydroxylieren kann. Estradiol ist aber nur unter Erzielung sehr geringer Ausbeuten an Verfahrensprodukt hydroxylierbar (US—A—3,179,674; siehe auch J. Org. Chem. *29,* 1964, 1333). 4,9-Estradien-3,17-dion ist sterisch gesehen ein ABC-Ringsystem ebenso planar wie das Estradiol und man sollte erwarten, daß es ebenfalls schlecht in 15α-Position zu hydroxylieren sei. Dies wird auch dadurch nahegelegt, weil nach den Beobachtungen von H. L. Holland et al. die bei Steroiden fast stets problemlos durchführbare 11α-Hydroxylierung bei diesem Substrat nicht gelingt (Can. J. Chem. *63,* 1985, 1121).

Die 15α-Hydroxylierung des Substrates wird unter den Bedingungen durchgeführt, die man üblicherweise bei der mikrobiologischen Hydroxylierung von Steroiden mit Pilzkulturen anwendet. So werden zunächst in allgemein üblichen Vorversuchen die günstigsten Fermentationsbedingungen, wie zum Beispiel Auswahl des günstigsten Nährmediums, des geeigneten Substratlösungs- oder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt.

Es ist zweckmäßig, das Substrat in einer Konzentration von etwa 100 bis 2000 mg pro Liter Nährmedium einzusetzen. Der pH-Wert wird vorzugsweise auf einen Bereich von 5 bis 7 eingestellt. Die Züchtungstemperatur liegt im Bereich von 20 bis 40°C, vorzugsweise von 25 bis 35°C. Zur Belüftung werden vorzugsweise 0,5 bis 5 Liter Luft pro Minute pro Liter Kulturbrühe zugeführt. Die Umwandlung des Substrats wird zweckmäßigerweise durch dünnschichtchromatographische Analyse verfolgt. Die Fermentationszeit beträgt etwa 15 bis 40 Stunden.

Führt man die sich gegebenenfalls anschließende Dehydratisierung des 15α-Hydroxy-4,9-estradien-3,17-dions unter den üblicherweise verwendeten Bedingungen durch (J. Fried und J. A. Edwards "Organic Reactions in Steroid Chemistry"; van Nostrand Reinhold Comp., New York, Vol. 1, 1972, p 320ff), so erhält man Gemische von 4,9,14- und 4,9,15-Estratrien-3,17-dion. Selektiv kann man das 4,9,14-Estratrien-3,17-dion darstellen wenn man die 15β-Hydroxyverbindung mit Phenylsulfenylchlorid (M. Fieser and L. Fieser: "Reagents for Organic Synthesis": John Wiley and Sons; New York, Vol. 5, 1975, p 523f) verestert und die Phenylsulfenylgruppe mittels Basen abspaltet. Eine geeignete Base ist beispielsweise Triethylamin, N-Methylpiperidin oder N-Methylmorpholin. Es ist nicht erforderlich, den Phenylsulfenylester zu isolieren sondern man kann ihn direkt abspalten.

Wenn man das 15α-Hydroxy-4,9-estratrien mit Trimethylacetanhydrid oder Trimethylacetylchlorid in Gegenwart von Basen vorzugsweise 4-Dimethylaminopyridin umsetzt, erhält man vorzugsweise das

4,9,15-Estratrien-3,17-dion. Auch hier ist es nicht erforderlich, die intermediär gebildeten Trimethylacetylverbindung zu isolieren.

Die sich gegebenenfalls anschließende Alkylierung des 4,9,15-Estratrien-3,17-dions erfolgt unter Bedingungen wie sie dem Fachmann wohl bekannt sind. M. Fieser, L. Fieser "Reagents for Organic Synthesis", Vol. 2, Seiten 151 bis 153, (J. Am. Chem. Soc., *103*, 7672, 1981).

Die so hergestellten 15β-Alkyl-4,9-estradien-3,17-dione sind wertvolle Zwischenprodukte zur Herstellung pharmakologisch wirksamer Steroidhormone. So kann man beispielsweise diese Verbindungen nachdem im Bull. Soc., Chem. France, 1970, 2548 ff. und 2556 ff. beschriebenen Verfahren in die entsprechenden Verbindungen der Formel IV

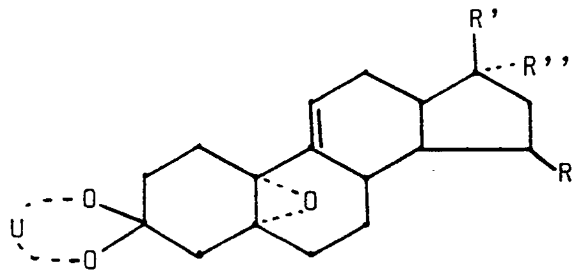

(IV),

oder Formel V

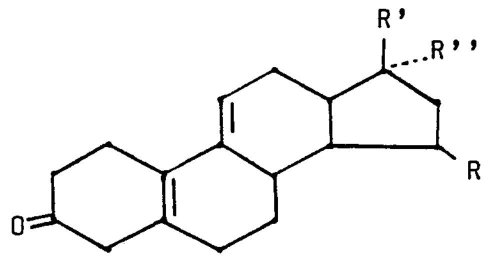

(V),

überführen worin R die eingangs genannte Bedeutung besitzt U eine Alkylengruppe mit 2 bis 6 Kohlenstoffatomen, R′ eine freie veresterte oder sililierte Hydroxygruppe R″ eine Cyanogruppe oder eine 1 bis 4 Kohlenstoffatome enthaltende aliphatische Kohlenwasserstoffgruppe darstellen.

Die so erhaltenen Verbindungen der allgemeinen Formel IV können in analoger Weise wie dies in der deutschen Offenlegungsschrift 29 20 184, in der europäischen Patentanmeldung 57115, in Tetrahedron Letters *22*, 1971, 2005, oder Tetrahedron Letters *22*, 1979, 2051 beschriebenen Weise in 15β-alkylierte Steroide überführt werden, welche die gleiche pharmakologische Wirkungsrichtung besitzen wie die entsprechenden 16-alkylierten Steroide oder die in 15-Position unsubstituierten Steroide.

Die nachfolgenden Ausführungsbeispiele dienen zur Erläuterung des erfindungsgemäßen Verfahrens.

### Beispiel 1

500 ml einer sterilen Nährlösung folgender Zusammensetzung:

3% Glucose, 1% Cornsteep, 0,2% Natriumnitrat, 0,1% Kaliumdihydrogenphosphat, Dikaliumhydrogenphosphat, 0,05% Magnesiumsulfat, 0,002% Eisen(II)-sulfat, 0,05% Kaliumchlorid werden mit einer 10 Tage alten Schrägagarkultur von Fasarium oxysporum (ATCC 7808) beimpft und 2½ Tage bei 30°C geschüttelt. Diese Vorkultur wird zur Beimpfung eines 20 l — Vorfermenters verwendet, der mit 15 l eines sterilen Mediums beschickt ist. Die Zusammensetzung des Mediums ist die gleiche wie oben angegeben. Der pH-Wert beträgt 6,0.

Die Vorkultur wird im Vorfermenter 24 Stunden unter Rühren (220 U/Min) und Belüftung (15 l/Min) bei 29°C weiter entwickelt.

Je 900 ml einer so erhaltenen Vorkultur werden in je einen 20 l — Hauptfermenter überführt, dessen Inhalt aus 14,1 l sterilen Mediums besteht.

Die Medien-Zusammensetzung:

3% Glucose, 1% Cornsteep, 0,2% Natriumnitrat, 0,1% Kaliumdihydrogenphosphat, 0,2% Dikaliumhydrogenphosphat, 0,05% Magnesiumsulfat, 0,002% Eisen(II)-sulfat, 0,05% Kaliumchlorid. Der pH-Wert beträgt 6,15.

Unter Rühren bei 220 U/Min. und Belüftung von 15 l Luft/Min bei 29°C wird die Kultur im Hauptfermenter weiter entwickelt. Nach 12 Stunden erfolgt die Substratzugabe.

7,5 g 4,9(10)-Estradien-3,17-dion werden in 100 ml N,N-Dimethylformamid gelöst, steril filtriert und zur Kultur zugegeben. Die Substratkonzentration beträgt 500 mg/l. Die Belüftung wird auf 5 l Luft/Min eingestellt und es wird weiter fermentiert.

Die Kontrolle der Umsetzung erfolgt mittels Dünnschichtchromatographie auf Fertigplatten Kieselgel 60 F 254 Merck.

Nach vollständiger Umwandlung des Ausgangsproduktes (24 Stunden Kontaktzeit) werden die Fermenteransätze vom Mycel abfiltriert. Das Filtrat und das Mycel werden mit Methylisobutylketon extrahiert. Die Extrakte werden vereinigt und im Vakuum bei 30 bis 35°C eingeengt. Der Rückstand wird zur Entfernung des Antischaummittels, Silikon SH, mit Hexan ausgewaschen. Das Rohprodukt wird über eine Kieselgelsäule mit Methylenchlorid/Ethanol chromatographiert. Die entsprechenden Fraktionen werden zusammengefaßt und aus Essigester umkristallisiert.

Bei einem Gesamteinsatz von 66 g Ausgangsprodukt wurden 19,5 g 15α-Hydroxy-4,9(10)-estradien-3,17-dion mit Schmelzpunkt 156 bis 157°C erhalten. Ausbeute: 27,9% der Theorie.

Als weiteres Umsetzungsprodukt wurde die dihydroxylierte Verbindung 11β,15α-Dihydroxy-4,9(10)-estradien-3,17-dion gefunden. 8,4 g = 11,3% der Theorie mit Schmelzpunkt 147/149 bis 151°C.

Beispiel 2

Unter den Bedingungen des Beispiel 1 werden 30,82 g 4,9(10)-estradien-3,17-dion bei einer Substratkonzentration von 250 mg/l fermentiert. Die Kontaktzeit beträgt 22 Stunden. Die Ausbeute an 15α-Hydroxy-4,9(10)-estradien-3,17-dion beträgt 10 g = 30,6% der Theorie mit Schmelzpunkt 157/158 bis 161°C.

An dihydroxylierter Verbindung entstehen 4,6 g = 13,3% der Theorie 11β,15α-Dihydroxy-4,9(10)-estradien-3,17-dion mit Schmelzpunkt 146/147 bis 149°C.

Beispiel 3

Unter den Bedingungen des Beispiel 1 werden 30 g 4,9(10)-estradien-3,17-dion bei einer Substratkonzentration von 400 mg/l fermentiert. Die Kontaktzeit beträgt 22 Stunden. Die Ausbeute an 15α-Hydroxy-4,9(10)-estradien-3,17-dion beträgt 10,3 g = 32,3% der Theorie mit Schmelzpunkt 158/159 bis 160°C.

An dihydroxylierter Verbindung entstehen 4,3 g = 12,8% der Theorie 11β,15α-Dihydroxy-4,9(10)-estradien-3,17-dion mit Schmelzpunkt 146/147 bis 149°C.

Beispiel 4

Unter den Bedingungen des Beispiel 1 werden 27 g 4,7,9(10)-estradien-3,17-dion bei einer Substratkonzentration von 650 mg/l fermentiert. Die Kontaktzeit beträgt 24½ Stunden. Die Ausbeute an 15α-Hydroxy-4,9(10)-estradien-3,17-dion beträgt 9 g = 31,4% der Theorie mit Schmelzpunkt 158/159 bis 160°C.

An dihydroxylierter Verbindung entstehen 3,4 g = 11,2% der Theorie 11β,15α-Dihydroxy-4,9(10)-estradien-3,17-dion mit Schmelzpunkt 146/147 bis 149°C.

Beispiel 5

Unter den Bedingungen des Beispiels 1 werden 7,05 g 4,9(10)-estradien-3,17-dion mit dem Stamm Glomerella Fusaroides ATCC 9552 bei einer Substratkonzentration von 250 mg/l fermentiert. Die Kontaktzeit beträgt 17½ Stunden.

Die Ausbeute an 15α-Hydroxy-4,9(10)-estradien-3,17-dion beträgt 1,59 g = 21,2% der Theorie mit Schmelzpunkt 146/148 bis 150°C.

11β,15α-Dihydroxy-4,9(10)-estradien-3,17-dion 0,07 g = 0,9% der Theorie mit Schmelzpunkt 140/141 bis 142°C.

Beispiel 6

4,9,15-Estratrien-3,17-dion

Eine Suspension von 19,5 g 15α-Hydroxy-4,9-estradien-3,17-dion in 300 ml Toluol wird unter Eiswasserkühlung skzessive mit 26,5 g Pivalinsäureanhydrid und 18,6 g 4-Dimethylaminopyridin versetzt. Anschließend rührt man 72 Stunden bei Raumtemperatur, gießt die Reaktionslösung danach in gesättigter Natriumhydrogencarbonat-Lösung, rührt weitere 30 Minuten bei Raumtemperatur und extrahiert mit Essigester. Die Essigesterextrakte werden mit gesättigter Ammoniumchlorid-Lösung und gesättigter Kochsalz-Lösung gewaschen, über Natriumsulfat getrocknet und im Wasserstrahlvakuum eingeengt. Das so erhaltene Rohprodukt wird über Aluminiumoxid, Merck, neutral, Stufe III mit Hexan/Essigester filtriert. Die Hauptfraktion wird aus Essigester kristallisiert. Man erhält 14,4 g (79% der Theorie) 4,9,15-Estratrien-3,17-dion vom Schmelzpunkt 182 bis 183°C.

Beispiel 7

15β-Methyl-4,9-estradien-3,17-dion

5,2 ml einer 5 %igen Lösung von Methyllithium in Diethylether werden bei −5°C portionsweise mit 570 mg Kupfer(I)-iodid versetzt. Man rührt bei einer Temperatur zwischen −5° und 0°C bis zur vollständigen Auflösung des Kupferiodids. Anschließend kühlt man auf −20°C und tropft eine Lösung von 1,1 g 4,9,15-Estratrien-3,17-dion in 15 ml absolutem Tetrahydrofuran langsam hinzu. Man rührt 20 Minuten bei −20°C, gießt anschließend in konzentrierte wässige Ammoniak-Lösung und extrahiert mit Essigester. Nach Chromatographie über Kieselgel mit Hexan/Essigester und Kristallisation der Hauptfraktion aus Essigester/Diisopropylether erhält man 720 mg 15β-Methyl-4,9-estradien-3,17-dion vom Schmelzpunkt 125 bis 126°C.

Beispiel 8

15β-n-Butyl-4,9-estradien-3,17-dion

Zu einer Suspension von 500 mg Kupfer(I)-cyanid in 10 ml absolutem Tetrahydrofuran tropft man bei −78°C 6,25 ml einer 15 %igen Lösung von n-Butyllithium in Hexan. Nach Zugabe rührt man weitere 15

Minuten bei −78°C, ehe eine Lösung von 1,2 g 4,9,15-Estratrien-3,17-dion in 20 ml absolutem Tetrahydrofuran tropfenweise hinzugegeben wird. Anschließend rührt man 90 Minuten, wobei man die Temperatur der Reaktionslösung allmählich auf −20°C ansteigen läßt. Zur Aufarbeitung gießt man in konzentrierte wässrige Ammoniak-Lösung und extrahiert mit Essigester. Nach Umkristallisation des Rohprodukts aus Essigester/Diisopropylether erhält man 940 mg 15β-n-Butyl-4,9-estradien-3,17-dion vom Schmelzpunkt 159 bis 161°C.

Beispiel 9

4,9,14-Estratrien-3,17-dion

Eine Lösung von 1,5 g 15α-Hydroxy-4,9-estradien-3,17-dion in 20 ml Methylenchlorid und 1,2 ml Triethylamin wird bei −20°C tropfenweise mit einer Lösung von 0,83 g Phenylsulfenylchlorid in 5 ml Methylenchlorid versetzt. Nach Zugabe rührt man 10 Minuten bei −20°C, verdünnt die Reaktionslösung anschließend mit 100 ml Methylenchlorid, wäscht nacheinander mit gesättigter Natriumhydrogencarbonat-Lösung und gesättigter Ammoniumchlorid-Lösung, trocknet über Natriumsulfat und engt ein. Kristallisation des Rohprodukts aus Essigester/Diisopropylether ergibt 1,24 g (88,2% der Theorie) 4,9,14-Estratrien-3,17-dion vom Schmelzpunkt 143 bis 144°C.

**Patentanspruch**

Verfahren zur Herstellung von Estran-Derivaten der allgemeinen Formel I

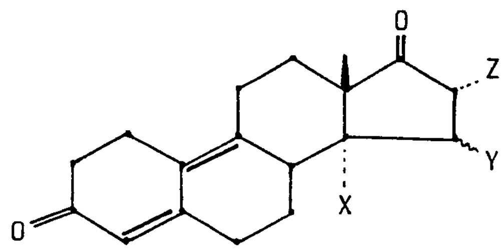

(I),

worin einer der Substituenten X oder Z ein Wasserstoffatom bedeutet und der andere gemeinsam mit Y eine Kohlenstoff-Kohlenstoffbindung darstellt, oder worin die Substituenten X und Z Wasserstoffatome darstellen und Y eine α-ständige Hydroxygruppe oder eine β-ständige Alkylgruppe mit bis zu 6 Kohlenstoffatomen darstellt, dadurch gekennzeichnet, daß man 4,9-Estradien-3,17-dion mit einer Pilzkultur von Fusarium oxysporum, Glomerella glycines, Glomerella fusaroides, Absidia coerulea fermentiert und gewünschtenfalls das erhaltene 15α-Hydroxy-4,9-estradien-3,17-dion in den Trimethylessigsäureester oder den Phenylsulfenylester überführt, die Trimethylacetylgruppe oder die Phenylsulfenylgruppe mittels Basen abspaltet sowie gegebenenfalls das erhaltene 4,9,15-Estratrien-3,17-dion mit einer Kupferalkylverbindung der allgemeinen Formel II oder III

$R_2CuLi$ (II) oder $R_2Cu(CN)Li_2$ (III),

worin R einen Alkylrest mit bis zu 6 Kohlenstoffatomen darstellt, alkyliert.

**Revendication**

Procédé pour préparer des dérivés de l'estrane qui répondent à la formule générale I:

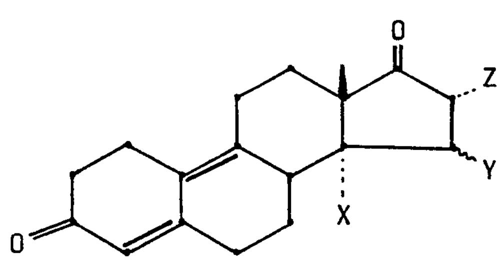

(I),

dans laquelle l'un des substituants X et Z représente un atome d'hydrogène et l'autre forme, avec Y, une liaison carbone-carbone, ou dans laquelle les substituants X et Z représentent chacun un atome d'hydrogène et Y représente un radical hydroxy avec la configuration α ou un radical alkyle avec la configuration β qui contient au plus 6 atomes de carbone, procédé caractérisé en ce qu'on fait fermenter l'estradiène-4,9 dione-3,17 avec une culture mycétienne de Fusarium oxysporum, de Glomerella glycines, de Glomerella fusaroides ou d'Absidia coerulea et, si on le désire, on transforme l'hydroxy-15α estradiène-

4,9 dione-3,17 obtenue en son ester triméthylacétique et en son ester phénylsulfénique, on coupe le radical triméthylacétyle ou phényl-sulfényle à l'aide de bases et, éventuellement, on alkyle l'estratriène-4,9,15 dione-3,17 obtenue au moyen d'un composé d'alkyl-cuivre répondant à l'une des formules générales II et III:

$R_2CuLi$ (II) $R_2Cu(CN)Li_2$ (III),

dans lesquelles R représente un radical alkyle contenant au plus 6 atomes de carbone.

**Claim**

Process for the manufacture of oestrane derivatives of the general formula I

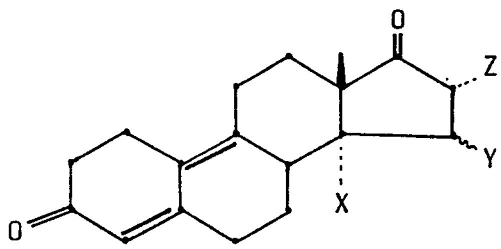

(I),

wherein one of the substituents X and Z represents a hydrogen atom and the other, together with Y, represents a carbon-carbon bond, or wherein the substituents X and Z represent hydrogen atoms and Y represents a hydroxy group in the α-configuration or an alkyl group in the β-configuration having up to 6 carbon atoms, characterised in that 4,9-oestradiene-3,17-dione is fermented with a fungus culture of Fusarium oxysporum, Glomerella glycines, Glomerella fusaroides or Absidia coerulea and, if desired, the resulting 15α-hydroxy-4,9-oestradiene-3,17-dione is converted into the trimethylacetic acid ester or the phenylsulphenyl ester, the trimethylacetyl group or the phenylsulphenyl group is split off using bases and, optionally, the resulting 4,9,15-oestratriene-3,17-dione is alkylated with an alkylcopper compound of the general formula II or III

$R_2CuLi$ (II) or $R_2Cu(CN)Li_2$ (III)

wherein R represents an alkyl radical having up to 6 carbon atoms.